Europäisches Patentamt

(19) European Patent Office

Office européen des brevets

(11) Publication number: **0 119 714**
**A2**

## (12) EUROPEAN PATENT APPLICATION

(21) Application number: **84300813.7**

(22) Date of filing: **09.02.84**

(51) Int. Cl.³: **A 61 B 17/32**, A 61 B 17/06

(30) Priority: **15.02.83 GB 8304129**

(43) Date of publication of application: **26.09.84**
Bulletin 84/39

(84) Designated Contracting States: **AT BE CH DE FR GB IT LI LU NL SE**

(71) Applicant: **HPW Limited, Concorde House Concorde Street, Luton Bedfordshire LU2 0JE (GB)**

(72) Inventor: **Hoskin, William John, 5 Long Buftlers, Harpenden Hertfordshire AL5 1JF (GB)**

(74) Representative: **Griffin, Kenneth David et al, Saunders & Dolleymore 2 Norfolk Road, Rickmansworth Hertfordshire WD3 1JH (GB)**

(54) Cutting and piercing devices.

(57) Cutting and piercing devices such as scalpels and needles for use in surgery and microsurgery are difficult to produce so as to be both sharp and durable.

Surprising and unexpected results were obtained by making the cutting and piercing regions of the devices of amorphous metals.

In one method a scalpel 12 of stainless steel was clamped in a heat sink 8 and immersed in a bath 2 liquid nitrogen (6). A laser beam (8) was caused to scan the cutting edge of the scalpel and to raise the local temperature to melting point and so transform the cutting edge to an amorphous state. As soon as the beam (8) had passed, extremely rapid cooling was effected to ensure that the amorphous condition of the cutting edge was substained.

# CUTTING AND PIERCING DEVICES

The present invention relates generally to devices for cutting and/or piercing, to methods and apparatus for manufacturing such devices and in particular to such devices for use in surgery and micro-surgery.

Traditionally, cutting blades and piercing needles for use in surgery are made primarily from stainless steel or titanium alloys, and although it is possible to achieve sharp, fine and relatively durable cutting edges and points respectively, such instruments in practice soon become sufficiently blunt to require regrinding.

The advent of surgical cutting instruments employing durable, finely ground diamond blades has had considerable success, but there still remain many blade sizes and/or shapes where the use of diamond material is not practical. Also, fixing such blades into canulae or on to metallic handles can be very difficult when the blades are small and/or when the blades have an unusual contour.

Thus, there is a need for a broad range of devices for cutting and/or piercing, particularly for employment in surgery, comprising conventional metallic materials, which are extremely sharp, very fine and durable.

In the present invention such a requirement has surprisingly and unexpectedly been achieved by the treatment of metallic materials with laser radiation.

Accordingly, the present invention provides a device for cutting or piercing comprising a metallic material in which the cutting edge or piercing point thereof is in an amorphous state.

The invention also provides a method of manufacturing a device for cutting or piercing comprising a metallic material in which the cutting edge or piercing point thereof is exposed to laser radiation to cause the structure of the material in the vicinity of the edge or point to become amorphous.

The expression "amorphous" is used to describe a homogeneous micro-structure in which the metallic material is in a glassy state without a regular crystal lattice.

The amorphous edge or point of the device after treatment with laser radiation is then finely ground to the required "sharp" condition by an appropriate honing procedure, using, for example, diamond pastes.

In order to achieve the most beneficial results, it is important to ensure that the laser "melts" and thereby renders amorphous no more than the cutting edge or point of the device being treated, and that heat generated within the material of the device is very rapidly conducted away from the cutting edge or point thereof, i.e. it is important to have a rapid chill rate of a thin molten zone.

Metallic materials suitable for treatment according to the present invention preferably comprise iron and titanium and alloys separately based on these materials. Stainless steel has been found to perform particularly well.

A $CO_2$ laser capable of rapidly scanning (e.g. at 0.5-5 cm/second) the treatment surface with a laser beam focussed to a power density in the range of 1-35 watts/cm$^2$ has been found to be particularly effective.

Advantageously, the conduction of heat from the cutting edge or point of the mass of the device is achieved by cooling the device to very low temperatures using cryogenic fluids, for example, liquid argon or liquid nitrogen ($T_b$ 77°K).

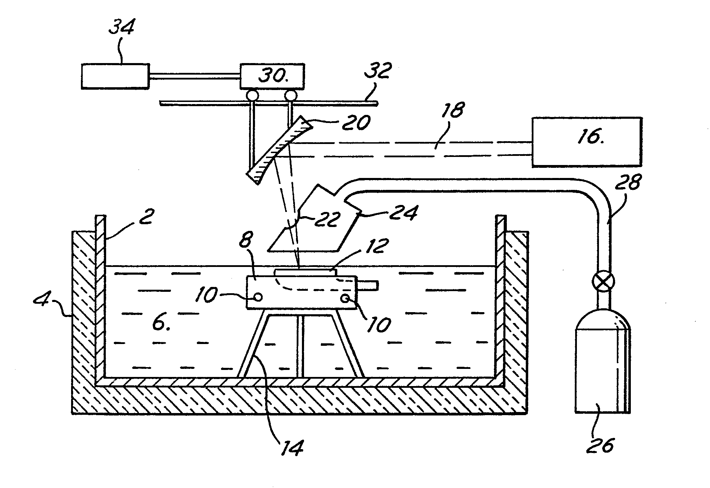

Apparatus for manufacturing a surgical knife will now be described by way of example with reference to the accompanying diagrammatic drawing in which the sole Figure is a cross-section through the apparatus.

As shown in the sole Figure a bath 2 with a thick thermally insulating jacket 4 is filled with liquid Nitrogen or Argon 6. A surgical stainless steel blade 12 having a cutting edge 10 mm long x 6.35 mm wide x 0.25 mm thick, is clamped between a pair of copper blocks 8 with the aid of screws or bolts 10. The assembly is then immersed in the bath where it is mounted on a support 14 in a manner such that the edge of the blade lies just below the surface of the liquid 6.

A 5 watt per cm$^2$ carbon dioxide laser 16 produces a laser beam 18 which is focussed by a mirror lens 20 through an opening 22 in a gas shield 24 on to the edge of the blade 12.

A reservoir 26 supplies an inert gas to the heat shield 24 via a conduit 28. The gas shield 24 operates to direct the inert gas to keep the blade edge shrouded in the inert gas so as to inhibit oxidation or other contamination.

The mirror lens 20 is supported by a carriage 30 mounted on rails 32 and reciprocated by a motor 34 at a rate of 1 cm per second.

In operation with the blade 12 clamped between the two copper blocks 8 and the blade edge lying just below the surface of the liquid 6 the laser is energised and the lens mirror displaced to displace the focussed laser beam along the edge of

-4-

the blade at a rate of 1 cm/sec. Any liquid 6 covering the blade edge evaporates fairly speedily and the temperature of the blade edge is temporarily raised to melting point. As soon as the focussed beam has passed, the molten metal is cooled extremely rapidly both due to the massive heat sinks provided by the copper blocks 8 and the low temperature of the liquid 6.

The rapid cooling ensures that the structure of the blade in the vicinity of its cutting edge becomes amorphous.

As soon as the whole of the cutting edge has been rendered amorphous it is removed from the bath 2 and the copper blocks, and finely ground to the required sharp condition by honing using diamond pastes.

It will be appreciated that instead of a blade of homogenous material the blade may be of composite structure with the body of the blade being of one material and the cutting region of the blade being formed by a coating of another material.

The coating can then be rendered amorphous by the above described method.

In a modification a blade may be provided with an amorphous coating by using evaporation or sputtering techniques.

Such procedures enable the body of the blade to be of almost any material for example a ceramic which could be ground to size and on to which the amorphous material would be deposited as a relatively thick (0.25 mm) coating. The blade is then lapped to produce a sharp cutting edge.

With this arrangement the amount of work involved to lap a cutting edge is much reduced since much less material needs to be removed.

0119714
830216

-5-

By making the coating sufficiently thick (in excess of 0.025 mm) lapping can readily be carried out and the adhesion produced between the coating and the substrate should be sufficient to prevent flaking or detachment during surgical sterilisation.

When using sputtering or evaporation techniques care must be taken to ensure fairly rapid cooling of the deposited coating to ensure that it remains amorphous.

The "sharpness" of a cutting edge or needle may be determined by conventional microscopic metallurgical examination.

The invention is applicable to both, parts for the manufacture of cutting and/or piercing devices, and to the resharpening of parts of existing devices.

## CLAIMS

1.      A cutting or piercing device characterised in that at least the cutting or piercing region of the device is a metal in an amorphous state.

2.      A device according to Claim 1 characterised in that the cutting or piercing region is rendered amorphous by heating to melting point and cooling sufficiently rapidly to sustain the amorphous state.

3.      A device according to Claim 1 characterised in that the cutting or piercing region is deposited as an amorphous coating by evaporation or sputtering techniques and then cooled sufficiently rapidly to sustain the amorphous state.

4.      A method of manufacturing a cutting or piercing device characterised by the steps of exposing the cutting edge or piercing point of a metallic surface to laser radiation to cause the structure of the material to become amorphous and then cooling the edge or point sufficiently rapidly to maintain it in its amorphous state.

5.      A method according to Claim 4 characterised by the step of clamping the device in a heat sink (8) and immersing the heat sink (8) and the device in a liquid coolant (6).

6.      A method according to Claim 5 characterised in that the coolant (6) is selected from the group consisting of liquid nitrogen and liquid argon.

7.      A method according to any one of Claims 4 to 6 characterised by the step of sharpening the amorphous cutting edge or point.

8.      A method of manufacturing a cutting or piercing device characterised by the steps of coating the sharp edge or point of a substrate with an amorphous metallic layer cooling the coating sufficiently rapidly to ensure the layer remains amorphous and sharpening the amorphous layer to a predetermined degree of sharpness.

9.      A method according to Claim 8 characterised in that said coating step is carried out by evaporation of the metal to form the coating.

10.      A method according to Claim 8 characterised in that the coating step is carried out by sputtering.

11.      Apparatus for rendering the cutting or piercing region of a cutting or piercing device (12) amorphous, the apparatus being characterised by a bath (2) for holding a liquid coolant (6), a heat sink (8) for clamping the device (12) and holding the cutting or piercing region of the device (12) just below the surface of the coolant (6), a laser (16) and a scanning arrangement (20,30 to 34) for causing the laser beam (18) produced by the laser (16) to scan the cutting or piercing region to render it amorphous.

12.      Apparatus according to Claim 11 characterised by an inert gas shield (24) for shrouding the cutting or piercing region in an inert gas to inhibit contamination thereof.

1/1